# EUROPEAN PATENT SPECIFICATION

(11) **EP 3 373 009 B1**
(45) Date of publication and mention of the grant of the patent: **01.09.2021**
(21) Application number: 17000369.3
(22) Date of filing: 07.03.2017
(51) Int. Cl.: G01N 33/574, G01N 1/30, G01N 33/58

(54) **FLUORESCENT COMPOUNDS FOR THE DETECTION OF MALIGNANT MELANOMA**
FLUORESZIERENDE VERBINDUNGEN ZUM NACHWEIS BÖSARTIGER MELANOME
COMPOSÉS FLUORESCENTS POUR LA DÉTECTION DE MÉLANOME MALIN

(43) Date of publication of application: 12.09.2018
(73) Proprietor: Karlsruher Institut für Technologie, 76131 Karlsruhe (DE)
(72) Inventor: Schepers, Ute, 53842 Troisdorf (DE); Bräse, Stefan, 53842 Troisdorf (DE); Olshausen, Bettina, 76131 Karlsruhe (DE)
(74) Representative: Müller-Boré & Partner Patentanwälte PartG mbB

(56) References cited:
- WO-A1-2015/124690
- US-A1- 2009 137 909
- US-A1- 2014 004 050
- US-A1- 2015 291 699
- XIN YAN ET AL: "Optical Imaging of Tumors with Copper-Labeled Rhodamine Derivatives by Targeting Mitochondria", THERANOSTICS, vol. 2, no. 10, 2012, pages 988-998, XP055405867,
- ZHUBAO ZHANG ET AL: "Integrin-Targeted Trifunctional Probe for Cancer Cells: A "Seeing and Counting" Approach", ANALYTICAL CHEMISTRY, vol. 84, no. 21, 2012, pages 8946-8951, XP055405956,
- KIM EUN J ET AL: "Optimizing the selectivity of DIFO-based reagents for intracellular bioorthogonal applications", CARBOHYDRATE RESEARCH, vol. 377, 2013, pages 18-27, XP028675595,
- TOBIAS HAGENDORN ET AL: "A Route to Cyclooct-2-ynol and Its Functionalization by Mitsunobu Chemistry", EUROPEAN JOURNAL OF ORGANIC CHEMISTRY, vol. 2014, no. 6, 2013, pages 1280-1286, XP055406810,
- PIERANGELO GOBBO ET AL: "Synthesis of a Toolbox of Clickable Rhodamine B Derivatives", SYNLETT, vol. 26, no. 09, 2015, pages 1169-1174, XP055406874,
- CHENG MA ET AL: "Fabrication of Versatile Cyclodextrin-Functionalized Upconversion Luminescence Nanoplatform for Biomedical Imaging", ANALYTICAL CHEMISTRY, vol. 86, no. 13, 2014, pages 6508-6515, XP055406911,
- M. CEKANOVA ET AL: "Molecular Imaging of Cyclooxygenase-2 in Canine Transitional Cell Carcinomas In Vitro and In Vivo", CANCER PREVENTION RESEARCH, vol. 6, no. 5, 2013, pages 466-476, XP055406905,
- M. J. UDDIN ET AL: "Selective Visualization of Cyclooxygenase-2 in Inflammation and Cancer by Targeted Fluorescent Imaging Agents", CANCER RESEARCH, vol. 70, no. 9, 2010, pages 3618-3627, XP055165595,
- LI GUO ET AL: "Synthesis and evaluation of a ligand targeting the somatostatin-2 receptor for drug delivery to neuroendocrine cancers", BIOORGANIC & MEDICINAL CHEMISTRY LETTERS, vol. 25, no. 8, 2015, pages 1792-1798, XP029155104,
- J UDDIN ET AL: "Design, Synthesis, and Structure-Activity Relationship Studies of Fluorescent Inhibitors of Cycloxygenase-2 as Targeted Optical Imaging Agents", BIOCONJUGATE CHEMISTRY, vol. 24, no. 4, 2013, pages 712-723, XP055138177,
- CEKANOVA MARIA ET AL: "Single-dose safety and pharmacokinetic evaluation of fluorocoxib A: pilot study of novel cyclooxygenase-2-targeted optical imaging agent in a canine model", INTERNATIONAL SOCIETY FOR OPTICAL ENGINEERING, vol. 17, no. 11, 2012, page 116002, XP060023743,
- JAAP DE LEEUW ET AL: "Fluorescence detection and diagnosis of non-melanoma skin cancer at an early stage", LASERS IN SURGERY AND MEDICINE., vol. 41, no. 2, 2009, pages 96-103, XP055407293,

## Description

The present invention relates to a pharmaceutical composition for use in an *in vivo* method for the detection of malignant melanoma, to an ex *vivo* method for the detection of malignant melanoma, and to a fluorescent compound for the detection of malignant melanoma.

Malignant melanoma is one of the most frequent and most dangerous forms of skin cancer. Accordingly, there is a considerable need for efficient diagnostic methods which allow to determine the presence of malignant melanoma particularly at an early stage so that it can be effectively treated. In particular, an early detection of malignant melanoma significantly enhances the survival of cancer patients.

However, the currently available methods for the detection of malignant melanoma are limited due to several aspects. The most common method in this respect is the visual inspection of the affected skin by a dermatologist and the evaluation of the lesions based on the so-called ABCDE rule (asymmetry, borders, color, diameter, and evolution over time). If a potential degeneration is observed, such as a pigment change, a tissue biopsy is carried out followed by a histological examination of the extracted tissue. The visual inspection of the affected skin represents an inexpensive method, but has the disadvantage of a low specificity. Moreover, repeated tissue biopsies may be required, thus being uncomfortable for the patient.

The specificity of the visual inspection can be increased when it is combined with dermatoscopy, whereby the number of necessary tissue biopsies to be carried out can be reduced. Besides the increased expenditure of time, a special training for the medical staff becomes mandatory when using a dermatoscope, which is a special type of an epiluminescence microscope.

Other methods for the detection of malignant melanoma known in the art include confocal microcopy, full body photography, electrical impedance spectroscopy, and new multispectral imaging methods such as MelaFind® and SIAscope.

An overview about the currently available methods for the detection of malignant melanoma can be taken from Mayer et al., J. Am. Acad. Dermatol. 2014, 71 (4), 599.e1-599.e12.

The above-mentioned methods for the detection of malignant melanoma are characterized by an improvement in the specificity and/or selectivity in comparison to the conventional visual inspection combined with dermatoscopy, but are associated with comparatively high costs and require a profound knowledge of the operator.

US 2009/137909 A1 describes a method for imaging and treating organs and tissues.

E. J. Kim et al., Carbohydr. Res. 2013, 377, 18-27 describes optimizing the selectivity of DIFO-based reagents for intracellular bioorthogonal applications.

C. Ma et al., Anal. Chem. 2014, 86(13), 6508-6515 describes the fabrication of a versatile cyclodextrin-functionalized upconversion luminescence nanoplatform for biomedical imaging.

Therefore, the technical problem underlying the present invention is to overcome the problems mentioned above and to provide an inexpensive and simple method for the detection of malignant melanoma with both a high specificity and a high selectivity.

This problem is solved by providing the embodiments characterized in the claims. In particular, according to a first aspect of the present invention, there is provided a pharmaceutical composition for use in an *in vivo* method for the detection of malignant melanoma. The pharmaceutical composition for use according to the present invention comprises a fluorescent compound for the detection of malignant melanoma (hereinafter also referred to as "fluorescent compound").

The chemical structure of the fluorescent compound disclosed herein is represented by the following general formula (I): 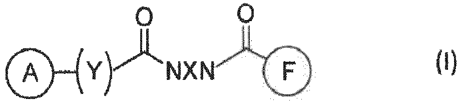 wherein
A represents a substituent selected from the group consisting of: 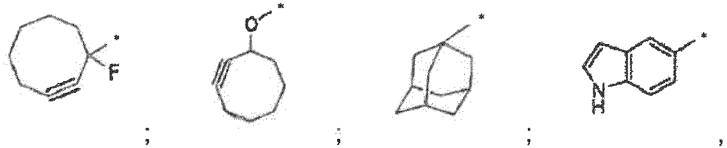
NXN represents a linear or cyclic C₁-C₈ diamine linker;
F represents a fluorescent dye selected from the group consisting of: 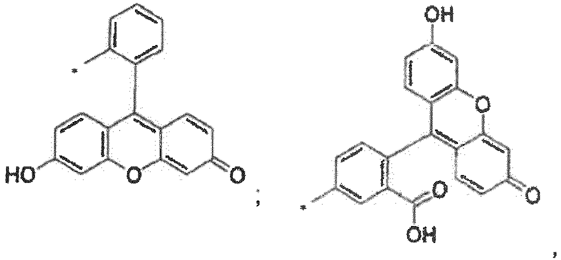 and
Y represents an optional C₁-C₁₂ spacer selected from the group consisting of a substituted or unsubstituted aliphatic moiety, a substituted or unsubstituted aromatic moiety, or a combination thereof.

As further outlined below, the fluorescent compound disclosed herein can be used for the detection of malignant melanoma, wherein the fluorescent compound, in form of the pharmaceutical composition comprising said fluorescent compound, is applied on a cutaneous tissue which is suspected of containing malignant melanoma cells.

The fluorescent compound for the detection of malignant melanoma disclosed herein is taken up by the malignant melanoma cells to a significantly higher extent compared to non-affected cells, e.g. healthy melanocytes. Accordingly, the fluorescent compound is effectively enriched in said malignant melanoma cells and can be detected by irradiation with a suitable light source. Thus, the fluorescent compound disclosed herein can be considered as a fluorescent probe for the detection of malignant melanoma.

Herein, the term "malignant melanoma" is understood as a type of cancer which arises from the melanin-producing melanocytes. When the melanocytes no longer respond to the normal control mechanisms of cellular growth, malignant melanoma develops, i.e. the healthy melanocytes turn into malignant melanoma cells. According to the present invention, malignant melanoma cells are limited to malignant melanoma cells occurring in the cutaneous tissue. In this respect, cutaneous tissue means the skin of a human or animal body.

Since the substituent A of the fluorescent compound disclosed herein is one of the above-depicted groups, the fluorescent compound is effectively taken up by the malignant melanoma cells without being enriched in non-affected cells, e.g. healthy melanocytes.

According to the present invention, the moiety NXN represents a linear or cyclic C₁-C₈ diamine linker in the general formula (I) of the above-defined fluorescent compound.

In a specific embodiment of the above-defined fluorescent compound, the diamine linker represented by NXN is selected from the group consisting of:

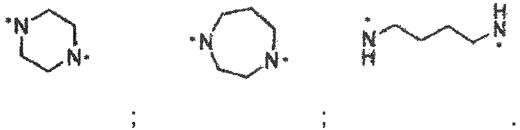

When the fluorescent compound disclosed herein contains one of the preferred diamine linkers depicted above, the fluorescent compound is effectively taken up by the malignant melanoma cells without being enriched in non-affected cells, e.g. healthy melanocytes.

The above-depicted fluorescent dyes F exhibit a high fluorescence quantum yield and have shown to be effectively enriched in malignant melanoma cells. Thereby, the detection of the fluorescence signal is facilitated, when using the fluorescent compound for the detection of malignant melanoma disclosed herein, the use of which is described in more detail below.

According to the present invention, the moiety Y in the general formula (I) of the above-defined fluorescent compound represents an optional C₁-C₁₂ spacer selected from the group consisting of a substituted or unsubstituted aliphatic moiety, a substituted or unsubstituted aromatic moiety, or a combination thereof.

In this context, the moiety Y may consist of carbon atoms and hydrogen atoms only, i.e. it may be unsubstituted. For example, the optional C₁-C₁₂ spacer may be a C₁-C₆ linear aliphatic moiety which may be saturated or unsaturated, i.e. may have one or more double and/or triple bonds. However, the moiety Y may also include one or more heteroatoms and/or may contain one or more cyclic or heterocyclic rings. According to the present invention, the spacer Y may be or may include a carbonyl group, an ether group, an alkylene group having 1 to 4 carbon atoms, which may be substituted with a hydroxy group, a mercapto group, an amino group and/or a halogen atom. The above-defined spacer Y may also be a combination of a cyclic or heterocyclic ring with one of the aforementioned groups. For example, the optional C₁-C₁₂ spacer may be derived from N-formyl-pyrrolidine. In another embodiment, the optional C₁-C₁₂ spacer is a phenylene moiety. In the fluorescent compound for the detection of malignant melanoma, the optional C₁-C₁₂ spacer Y may be absent or may be present as required.

Specific Examples of the above-defined fluorescent compound, comprising a specific substituent A, a specific diamine linker NXN, and a specific fluorescent dye F, along with an optional C₁-C₁₂ spacer Y in accordance with the present invention, include the structures (1-1), (I-14) (1-18), (I-20), (I-25), (I-26), (I-27) and (I-28) contained in Table A:

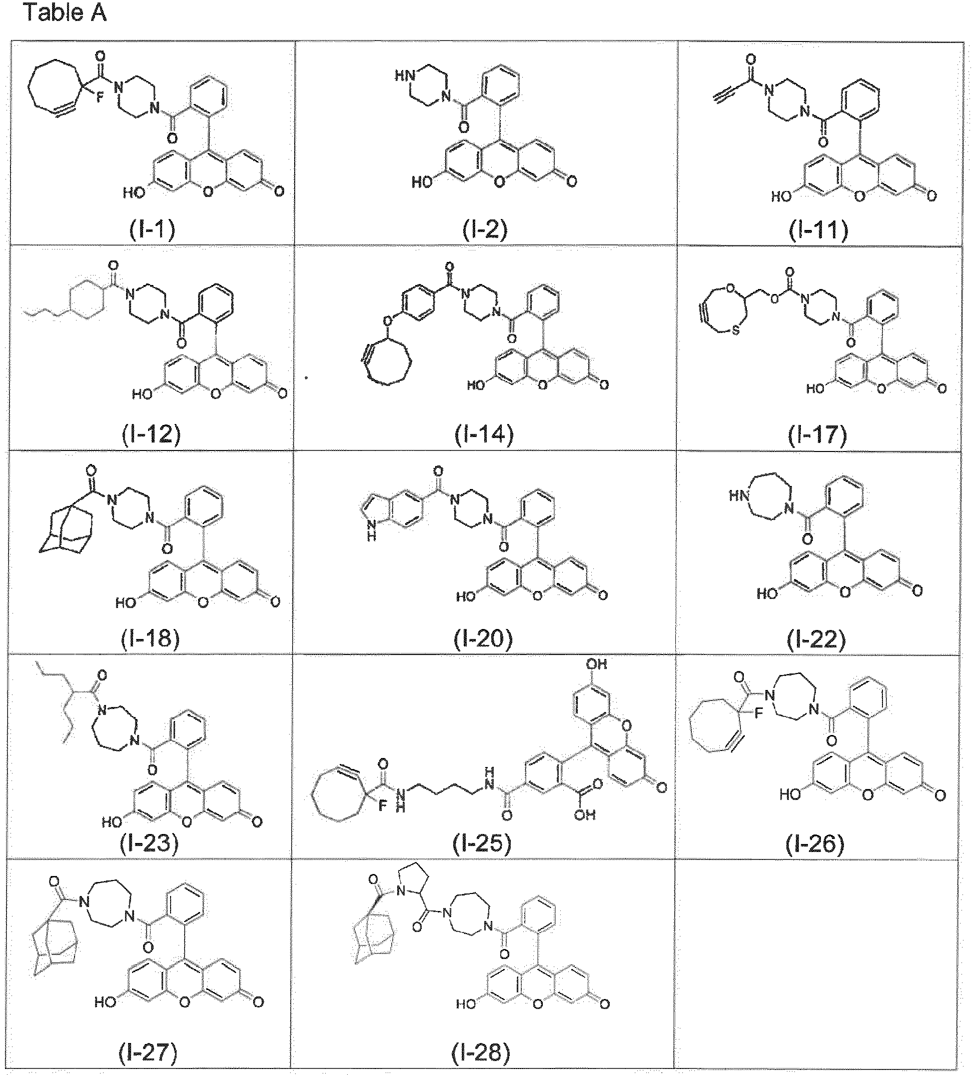

In a specific embodiment of the present invention, the fluorescent compound as defined above is selected from the group consisting of:

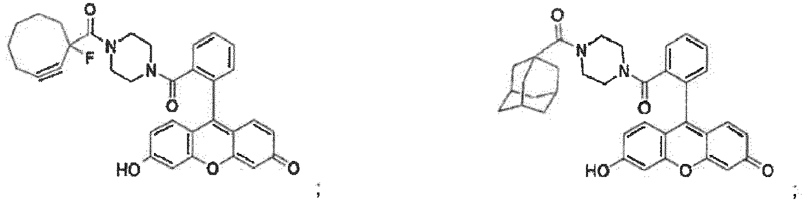

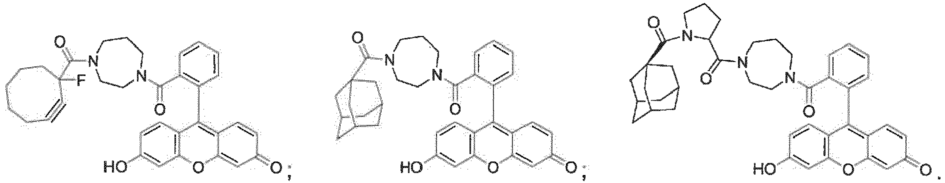

According to the present invention, the pharmaceutical composition is for use in an *in vivo* method for the detection of malignant melanoma (hereinafter also referred to as "pharmaceutical composition"). The pharmaceutical composition has the purpose of providing the above-defined fluorescent compound in a suitable dosage form, e.g. in a ready-to-use application form.

The pharmaceutical composition for use according to the present invention comprises the fluorescent compound as defined above, and optionally, a pharmaceutically acceptable excipient and/or one or more further additives. In the pharmaceutical composition, the pharmaceutically acceptable excipient as well as any additives may be absent or may be present as required.

The pharmaceutically acceptable excipient is not limited according to the present invention. It can, for example, be selected from a solvent such as water and ethanol, provided that it allows to solve or stably disperse the above-defined fluorescent compound.

According to the present invention, the mode of administration of the pharmaceutical composition is not specifically limited as long as it allows the fluorescent compound to be taken up by any potential melanoma cells. For example, the pharmaceutical composition may be administered in form of a spray, a lotion, a salve, a powder, a gel, etc. In one embodiment of the present invention, the above-defined pharmaceutical composition is provided as a spray or as a salve. In this case, the pharmaceutically acceptable excipient to be contained in the pharmaceutical composition is adjusted such that the desired dosage form is obtained.

In a specific embodiment of the above-defined pharmaceutical composition, the concentration of the fluorescent compound is not specifically limited and can be adjusted depending on the type of fluorescent compound of the present invention. For example, the concentration of the fluorescent compound in the pharmaceutical composition may be in the range from 0.1 µM to 100 µM, e.g. 0.5 µM to 50 µM, 1 µM to 10 µM, or 2 µM to 5 µM.

In a specific embodiment of the pharmaceutical composition for use in an *in vivo* method for the detection of malignant melanoma, the method may comprise the steps of:
(a) applying the above-defined pharmaceutical composition on a cutaneous tissue which is suspected of containing malignant melanoma cells;
(b) incubating the cutaneous tissue for a period of 1 min to 6 hrs;
(c) optionally removing any excess pharmaceutical composition from the cutaneous tissue;
(d) irradiating the cutaneous tissue by using an excitation light source which is suitable for exciting the fluorescent compound contained in the pharmaceutical composition;
(e) visually examining the cutaneous tissue; and
(f) based on the fluorescence signal obtained in step (e), determining the presence of malignant melanoma in the cutaneous tissue.

The above-defined pharmaceutical composition for use according to the present invention is not limited to the method for the detection of malignant melanoma comprising the steps (a) to (f) as defined above.

In another aspect of the present invention, there is provided an *ex vivo* method for the detection of malignant melanoma, comprising the steps (a) to (f) as defined above.

It is to be noted that the following definitions apply equally to the pharmaceutical composition for use in an *in vivo* method comprising steps (a) to (f), as defined above, and to the respective *ex vivo* method.

The *in vivo* and ex *vivo* method comprising steps (a) to (f) is described in detail hereinafter.

In step (a), the pharmaceutical composition comprising the fluorescent compound for the detection of malignant melanoma as defined above is applied on a cutaneous tissue which is suspected of containing malignant melanoma cells. As required, the cutaneous tissue to be examined may be extracted before the application of the above-defined pharmaceutical composition. Subsequent to the application, the fluorescent compound is selectively taken up by any malignant melanoma cells. Accordingly, non-affected cells, e.g. healthy melanocytes do not enrich the fluorescent compound as defined above.

In step (b), the cutaneous tissue which has been exposed to the pharmaceutical composition is incubated for a period of 1 min to 6 hrs after the application so that the fluorescent compound is effectively taken up by the malignant melanoma cells. This step allows to increase the concentration of the fluorescent compound in the malignant melanoma cells, thus facilitating the detection of the fluorescence signal in step (f). The period of incubating the cutaneous tissue depends on the amount of the pharmaceutical composition applied, but falls within the above-mentioned range. Specific incubation times include ranges of 10 min to 5 hrs, 20 min to 4 hrs, or 30 min to 3 hrs. For example, the incubation time is from 4 hrs to 5 hrs.

In step (c) which is optional, any excess of the above-defined pharmaceutical composition is removed from the exposed cutaneous tissue. For example, removal of the excess pharmaceutical composition can be carried out by washing off any residual pharmaceutical composition with a suitable solvent, such as water. As already mentioned above, step (c) is optional, and may be required only, e.g. in case an excess of the pharmaceutical composition disclosed herein is applied on the cutaneous tissue which is suspected of containing malignant melanoma cells.

In step (d), the cutaneous tissue which has been exposed to the above-defined pharmaceutical composition is irradiated with an excitation light source which is suitable for exciting the fluorescent compound contained in said pharmaceutical composition.

In this context, the emission wavelength of the excitation light source is not further limited, but has to match the absorption range of the fluorescent dye contained in the fluorescent compound for the detection of malignant melanoma. In addition, it is preferable that the excitation light source has a narrow emission range so as to avoid the excitation of any other constituents of the cutaneous tissue. Such an additional excitation could lead to an undesired fluorescence signal which might overlap with the fluorescence signal emitted by the fluorescent compound, thus being detrimental to the detection of malignant melanoma.

Suitable ranges of excitation wavelengths include e.g. 450 nm to 550 nm, 460 nm to 520 nm, 470 nm to 500 nm, or 480 nm to 495 nm. In this respect, it is clear to a person skilled in the art that the specific excitation wavelength depends on the fluorescent dye F in the above general formula (I).

Due to its ease of use and its low costs, a customary UV lamp may be used as the excitation light source in step (d).

In step (e), the cutaneous tissue irradiated with the excitation light source is visually examined. According to the present invention, the visual examination is not particularly limited. It can be carried out with the human eye or with a technical detection unit, for example, with an epifluorescence microscope equipped with a CCD camera. However, the use of a technical detection unit is not necessarily required in the method according to the present invention.

When visually examining the irradiated cutaneous tissue without using a technical detection unit, no specific training of the operator is required, and the above-defined method is particularly inexpensive.

In step (f), the presence of malignant melanoma is determined in the examined cutaneous tissue based on the fluorescence signal obtained in step (e). If a fluorescence signal is obtained, it has to be concluded that the examined cutaneous tissue contains malignant melanoma cells, since the fluorescent compound disclosed herein is almost exclusively taken up by malignant melanoma cells. A quantitative evaluation can principally be accomplished using microscopic techniques, e.g. by analyzing the signal-to-noise ratio.

In a further aspect of the present invention, there is provided a fluorescent compound for the detection of malignant melanoma, wherein the fluorescent compound is selected from the group consisting of:

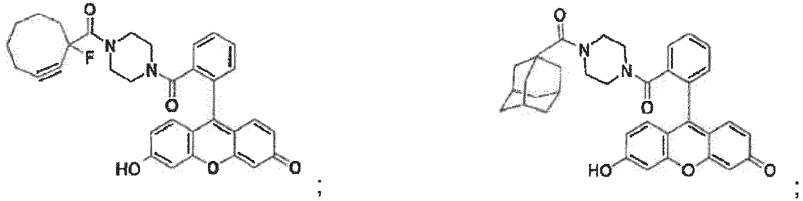

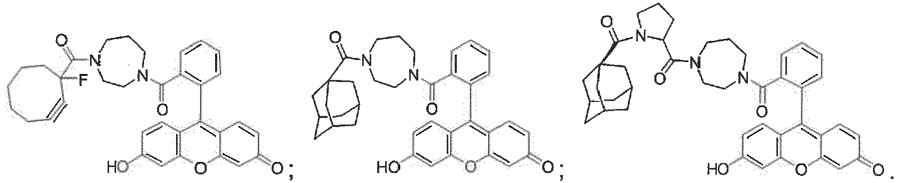

Due to the unique chemical structure of the fluorescent compound, it is specifically enriched in malignant melanoma cells so that a significant fluorescence signal is obtained when such cells are present in the examined cutaneous tissue. Accordingly, using the above-defined fluorescent compound allows to detect malignant melanoma with a high specificity, since the number of false positives tends to be low. Furthermore, the selectivity thereof is also high, since the number of false negatives tends to be low as well.

The present invention provides a simple and inexpensive method for the detection of malignant melanoma which neither requires a special training of the operator nor any elaborate technical equipment. Due to its high selectivity, even small lesions comprising clusters of malignant melanoma cells can be reliably detected. In addition, in view of its high specificity, using the above-defined fluorescent compound allows to effectively distinguish the malignant melanoma from other kinds of skin disease, so that time-consuming differential diagnosis can be reduced or avoided.

In contrast to the above-mentioned multispectral imaging methods known in the art, the fluorescence signal obtained in the method disclosed herein is directly generated from the above-defined fluorescent compound. Accordingly, the detection of malignant melanoma according to the present invention is not based on the fluorescence properties of the pigment melanin itself. Whereas melanin may be present both in non-affected cells, e.g. healthy melanocytes, as well as in malignant melanoma cells, the fluorescent compound as defined above is taken up by the malignant melanoma cells to a significantly higher extent compared to non-affected cells. Therefore, in the multispectral imaging methods known in the art, a complex evaluation of the recorded spectra or images is required in order to determine subtle differences in the fluorescence signal, rendering said methods costly and time-consuming. These shortcomings are overcome by using the fluorescent compound for the detection of malignant melanoma disclosed herein.

Hereinafter, the above-defined fluorescent compound for the detection of malignant melanoma is abbreviated as MSF ("melanoma specific fluorophore").

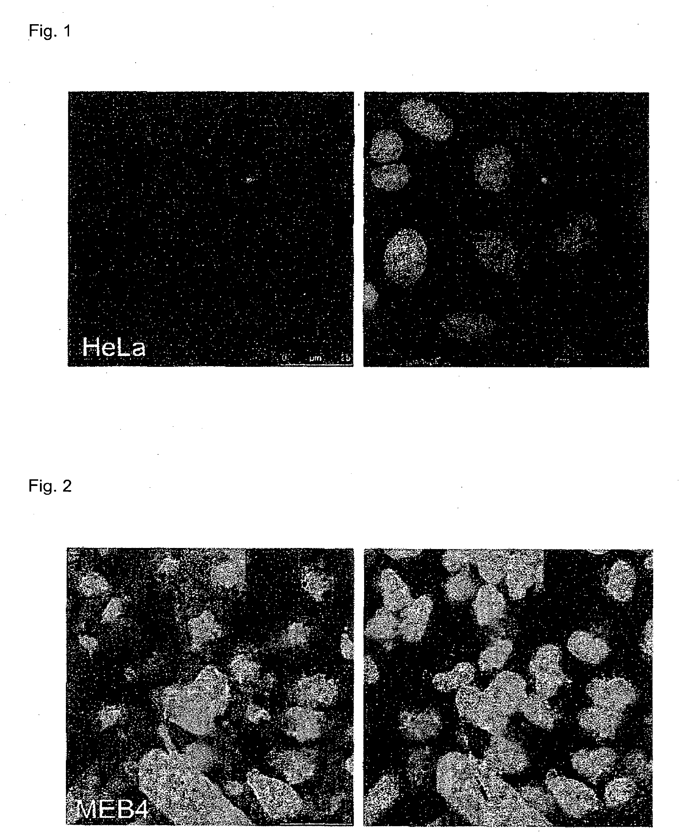

Fig. 1 shows two CLSM images of HeLa cells treated with the MSF in Example 1. In order to visualize the cell nuclei, the latter have been stained using 0.5 µg/mL Hoechst 33342 (Invitrogen) for 20 min after washing the cell layer three times with PBS (phosphate buffered saline), which can be taken from the image on the right side. As can be taken from both the left and the right side image, there is no fluorescence apparent which could originate from the MSF.

Fig. 2 shows two CLSM images of MEB4 cells treated with the MSF in Example 1. In order to visualize the cell nuclei, the latter have been stained using 0.5 µg/mL Hoechst 33342 (Invitrogen) for 20 min after washing the cell layer three times with PBS (phosphate buffered saline), which can be taken from the image on the right side. As can be taken from both the left and the right side image, fluorescence generated by the MSF can be recognized throughout the cytoplasm.

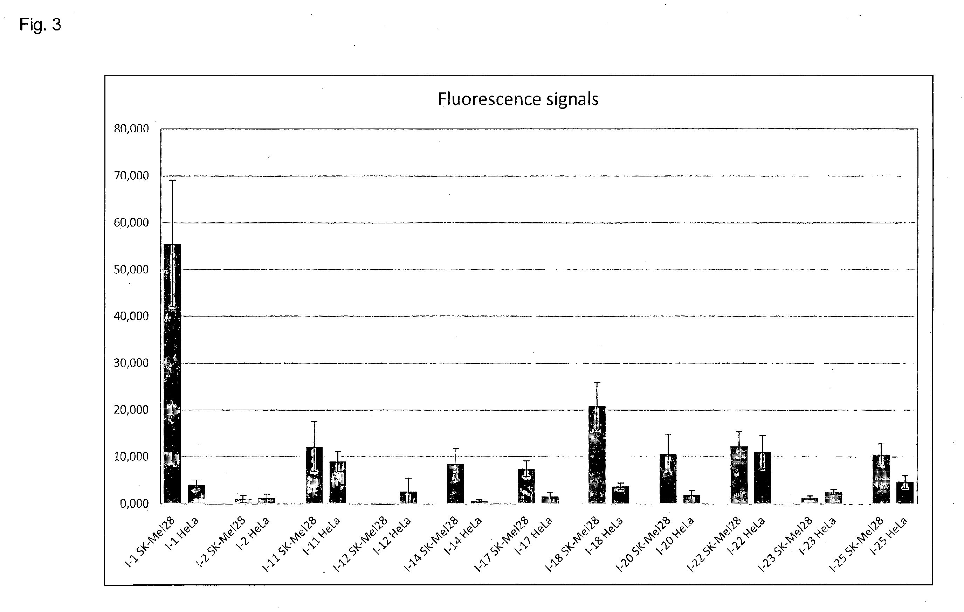

Fig. 3 shows the fluorescence signals of the fluorescent compounds used for detection of malignant melanoma in Example 2.

The following Examples are intended to further illustrate the present invention. The claims are not to be construed as being limited thereto.

### Example 1:

Two-dimensional cell cultures were treated with the fluorescent compound (1-1) as the MSF, represented by the following formula:

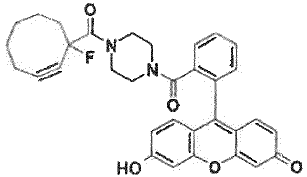

Diverse cell lines as well as primary skin cells such as U2OS, SHSY, A459, H4, Hek293, HepG2, HCT116, U251, MCF-7, Ratfib, HUVEC, Fibroblasts, and HeLa were treated with the MSF at a concentration of 20 µM in DMF (dimethylformamide) for 5 hrs and subsequently evaluated by means of CLSM (confocal laser scanning microscopy).

In addition, murine (MEB4) as well as human (SK-Mel28) malignant melanoma cells were treated with the MSF under the same conditions. The results are shown in Figs. 1 and 2. As can be taken from Fig. 1, the MSF has effectively been taken up by the MEB4 cells, i.e. by the malignant melanoma cells. In contrast thereto, the MSF has not significantly been taken up by the HeLa cells, i.e. by the non-affected cells.

Apart from the above-described qualitative analysis of the CLSM images, it is also possible to quantitatively evaluate said CLSM images by means of an automatized screening microscopy procedure.

The experimental procedure was similar to that of Example 2, as described below.

### Example 2:

The cellular uptake of the fluorescent compounds according to the present invention was studied by treatment of human melanoma cells SK-Mel28 and HeLa cells (human cervix carcinoma cell line as a negative control, common model cell line) and their investigation via CLSM (confocal laser scanning microscopy).

The aforementioned cells were seeded in an ibidi® µ-slide 8 well in a density of 2x10⁵/mL and cultured for two days to confluency, before being treated with the fluorescent compounds in a concentration of 20 µM in DMF (dimethylformamide). After an incubation time of 5 hrs, the adherent cell layers were washed three times with PBS (phosphate buffered saline) and the nuclei were stained with Hoechst 33342 (Invitrogen, 0.5 µg/mL, 20 min). During the microscopic studies (Leica TCS-SP-5, inverted microscope), the fluorescent compounds were excited using an Ar-laser at 488 nm. Excitation of the Hoechst dye was accomplished with an UV-Laser at 351 and 364 nm. Due to the overlap of the emission spectra of both dyes, a sequential mode was chosen for recording the cell images.

The resulting cell images were analyzed in terms of fluorescence quantification at 488 nm excitation using the software ImageJ 1.50i (Wayne Rasband, National Institutes of Health, USA).

From each cell image, five cells were selected as regions of interest and the related fluorescence signals were quantified (mean value and standard deviation). Additionally, three cell-free regions were selected in each cell image as background and the resulting background signal from these regions was subtracted from the cell signal.

Fig. 3 shows the evaluation of the fluorescence signals from fluorescent compounds (I-1), (I-2), (I-11), (I-12), (I-14), (1-17), (I-18), (I-20), (I-22), (I-23) and (I-25) in SK-Mel28 (human melanoma cells) and HeLa (human cervix carcinoma, control).

With a high fluorescence signal and a large difference in the uptake between SK-Mel28 and HeLa cells, fluorescent compound (I-1) showed the best results. Fluorescent compounds (I-14), (I-17)*, (I-18), (I-20) and (I-25) also showed significant differences in the uptake in different cell lines, but slightly weaker fluorescence signals (* not falling within the scope of the present invention).

## Claims

1. A pharmaceutical composition for use in an *in vivo* method for the detection of malignant melanoma in a cutaneous tissue, comprising a fluorescent compound for the detection of malignant melanoma, represented by the following general formula (I), and optionally a pharmaceutically acceptable excipient: 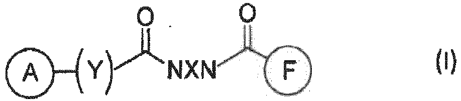 wherein
A represents a substituent selected from the group consisting of: 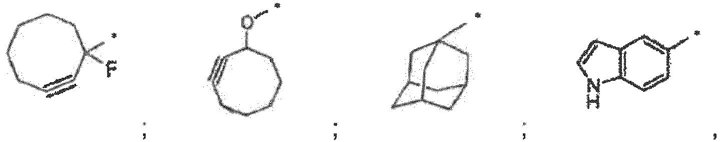
NXN represents a linear or cyclic C₁-C₈ diamine linker;
F represents a fluorescent dye selected from the group consisting of: 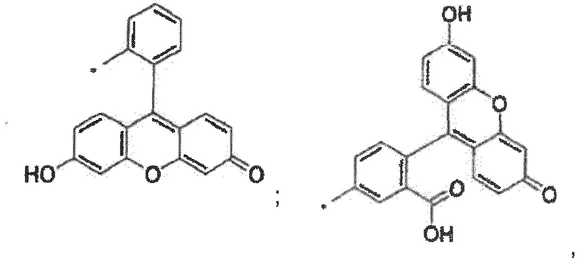 and
Y represents an optional C₁-C₁₂ spacer selected from the group consisting of a substituted or unsubstituted aliphatic moiety, a substituted or unsubstituted aromatic moiety, or a combination thereof.

2. The pharmaceutical composition for use according to claim 1, wherein the diamine linker NXN is selected from the group consisting of:

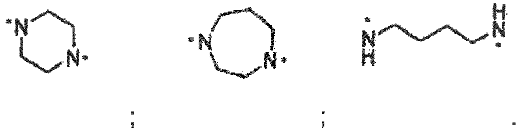

3. The pharmaceutical composition for use according to claim 1 or 2, wherein the fluorescent compound is selected from the group consisting of:

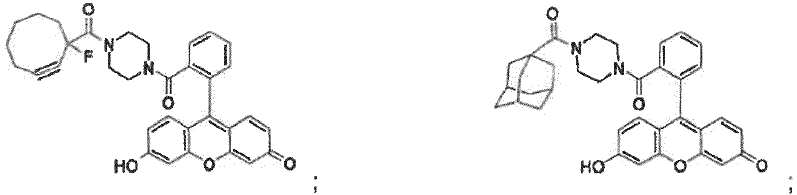

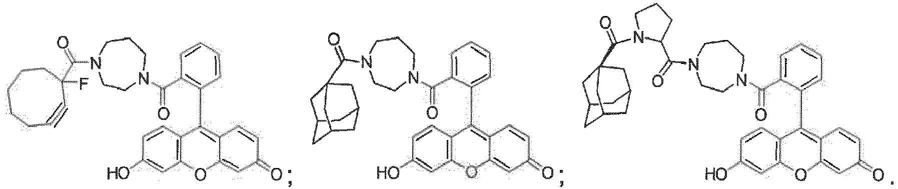

4. The pharmaceutical composition for use according to any one of claims 1 to 3, wherein the pharmaceutical composition comprises a pharmaceutically acceptable excipient and is a spray or a salve.

5. The pharmaceutical composition for use according to claim 4, wherein the concentration of the fluorescent compound is from 0.1 µM to 100 µM.

6. The pharmaceutical composition for use according to any one of claims 1 to 5, wherein the method comprises the steps of:
(a) applying the pharmaceutical composition on a cutaneous tissue which is suspected of containing malignant melanoma cells;
(b) incubating the cutaneous tissue for a period of 1 min to 6 hrs;
(c) optionally removing any excess pharmaceutical composition from the cutaneous tissue;
(d) irradiating the cutaneous tissue by using an excitation light source which is suitable for exciting the fluorescent compound contained in the pharmaceutical composition;
(e) visually examining the cutaneous tissue; and
(f) based on the fluorescence signal obtained in step (e), determining the presence of malignant melanoma in the cutaneous tissue.

7. The pharmaceutical composition for use according to claim 6, wherein the excitation light source in step (d) is a customary UV lamp.

8. An ex *vivo* method for the detection of malignant melanoma in a cutaneous tissue, wherein the method comprises the steps of:
(a) applying the pharmaceutical composition according to any one of claims 1 to 5 on a cutaneous tissue which is suspected of containing malignant melanoma cells;
(b) incubating the cutaneous tissue for a period of 1 min to 6 hrs;
(c) optionally removing any excess pharmaceutical composition from the cutaneous tissue;
(d) irradiating the cutaneous tissue by using an excitation light source which is suitable for exciting the fluorescent compound contained in the pharmaceutical composition;
(e) visually examining the cutaneous tissue; and
(f) based on the fluorescence signal obtained in step (e), determining the presence of malignant melanoma in the cutaneous tissue.

9. The ex *vivo* method according to claim 8, wherein the excitation light source in step (d) is a customary UV lamp.

10. A fluorescent compound for the detection of malignant melanoma, wherein the fluorescent compound is selected from the group consisting of:

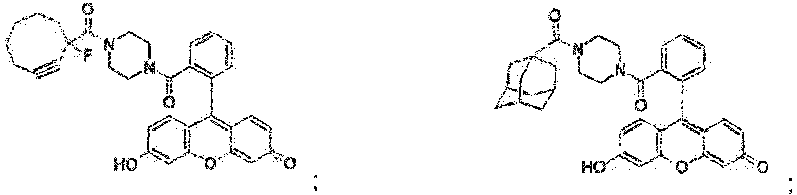

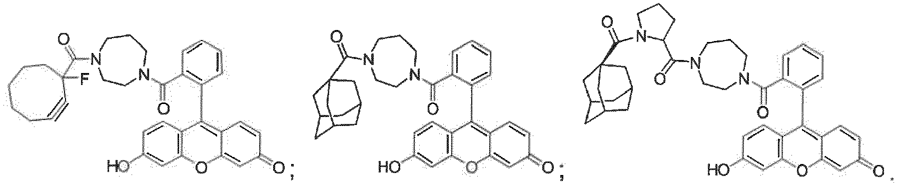

## Patentansprüche

1. Pharmazeutische Zusammensetzung zur Verwendung in einem *in vivo-*Verfahren zum Nachweis von malignem Melanom in einem Hautgewebe, umfassend eine fluoreszierende Verbindung zum Nachweis von malignem Melanom, dargestellt durch die nachstehende allgemeine Formel (I), und gegebenenfalls einen pharmazeutisch verträglichen Hilfsstoff: 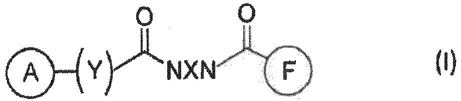 wobei
A einen Substituenten darstellt, ausgewählt aus der Gruppe, bestehend aus: 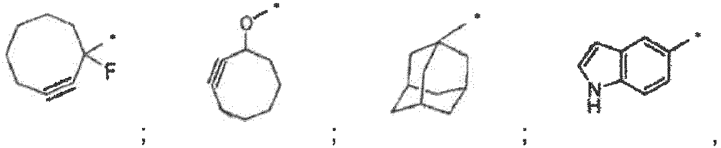
NXN eine unverzweigte oder zyklische C₁-C₈-Diaminverknüpfungsgruppe darstellt;
F einen fluoreszierenden Farbstoff darstellt, ausgewählt aus der Gruppe, bestehend aus: 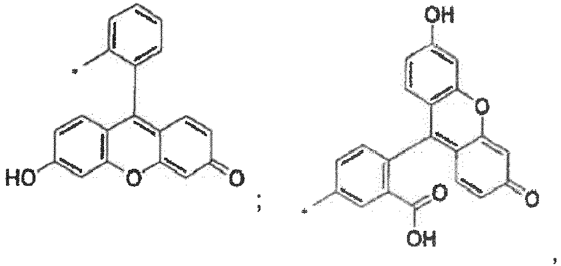 und
Y einen optionalen C₁-C₁₂-Abstandshalter darstellt, ausgewählt aus der Gruppe, bestehend aus einem substituierten oder unsubstituierten aliphatischen Rest, einem substituierten oder unsubstituierten aromatischen Rest, oder einer Kombination hiervon.

2. Pharmazeutische Zusammensetzung zur Verwendung nach Anspruch 1, wobei die Diaminverknüpfungsgruppe NXN aus der Gruppe ausgewählt ist, bestehend aus:

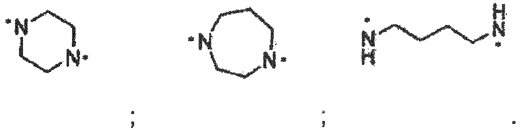

3. Pharmazeutische Zusammensetzung zur Verwendung nach Anspruch 1 oder 2, wobei die fluoreszierende Verbindung aus der Gruppe ausgewählt ist, bestehend aus:

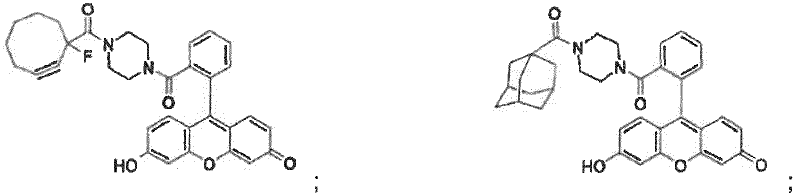

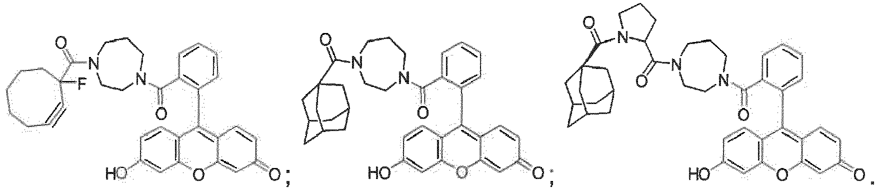

4. Pharmazeutische Zusammensetzung zur Verwendung nach einem der Ansprüche 1 bis 3, wobei die pharmazeutische Zusammensetzung einen pharmazeutisch verträglichen Hilfsstoff umfasst und ein Spray oder eine Salbe ist.

5. Pharmazeutische Zusammensetzung zur Verwendung nach Anspruch 4, wobei die Konzentration der fluoreszierenden Verbindung von 0,1 µM bis 100 µM beträgt.

6. Pharmazeutische Zusammensetzung zur Verwendung nach einem der Ansprüche 1 bis 5, wobei das Verfahren die Schritte umfasst:
(a) Auftragen der pharmazeutischen Zusammensetzung auf ein Hautgewebe, welches im Verdacht steht, maligne Melanomzellen zu enthalten;
(b) Inkubieren des Hautgewebes für eine Dauer von 1 Min. bis 6 Std.;
(c) gegebenenfalls Entfernen überschüssiger pharmazeutischer Zusammensetzung von dem Hautgewebe;
(d) Bestrahlen des Hautgewebes unter Verwendung einer Anregungslichtquelle, welche zur Anregung der fluoreszierenden Verbindung, enthalten in der pharmazeutischen Zusammensetzung, geeignet ist;
(e) visuelles Begutachten des Hautgewebes; und
(f) basierend auf dem in Schritt (e) erhaltenen Fluoreszenzsignal, Bestimmen, ob malignes Melanom in dem Hautgewebe vorliegt.

7. Pharmazeutische Zusammensetzung zur Verwendung nach Anspruch 6, wobei die Anregungslichtquelle in Schritt (d) eine handelsübliche UV-Lampe ist.

8. ex *vivo*-Verfahren zum Nachweis von malignem Melanom in einem Hautgewebe, wobei das Verfahren die Schritte umfasst:
(a) Auftragen der pharmazeutischen Zusammensetzung nach einem der Ansprüche 1 bis 5 auf ein Hautgewebe, welches im Verdacht steht, maligne Melanomzellen zu enthalten;
(b) Inkubieren des Hautgewebes für eine Dauer von 1 Min. bis 6 Std.;
(c) gegebenenfalls Entfernen überschüssiger pharmazeutischer Zusammensetzung von dem Hautgewebe;
(d) Bestrahlen des Hautgewebes unter Verwendung einer Anregungslichtquelle, welche zur Anregung der fluoreszierenden Verbindung, enthalten in der pharmazeutischen Zusammensetzung, geeignet ist;
(e) visuelles Begutachten des Hautgewebes; und
(f) basierend auf dem in Schritt (e) erhaltenen Fluoreszenzsignal, Bestimmen, ob malignes Melanom in dem Hautgewebe vorliegt.

9. ex *vivo*-Verfahren nach Anspruch 8, wobei die Anregungslichtquelle in Schritt (d) eine handelsübliche UV-Lampe ist.

10. Fluoreszierende Verbindung zum Nachweis von malignem Melanom, wobei die fluoreszierende Verbindung aus der Gruppe ausgewählt ist, bestehend aus:

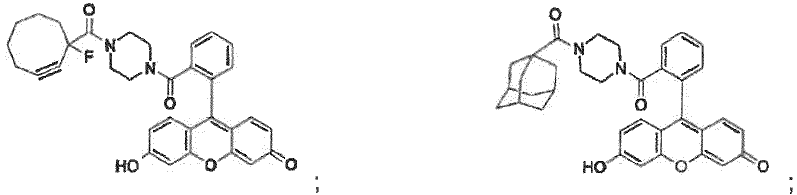

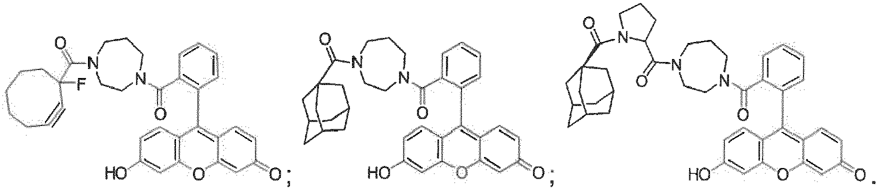

## Revendications

1. Composition pharmaceutique destinée à être utilisée dans un procédé in vivo pour la détection d'un mélanome malin dans un tissu cutané, comprenant un composé fluorescent pour la détection d'un mélanome malin, représenté par la formule générale suivante (I), et en option un excipient pharmaceutiquement acceptable : 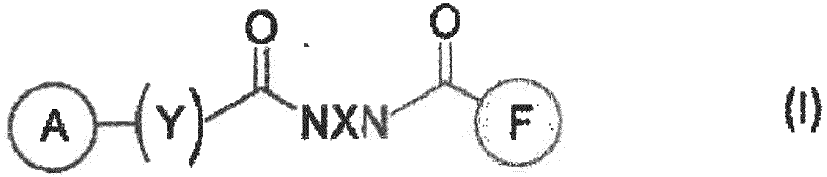 dans laquelle
A représente un substituant sélectionné parmi le groupe constitué de : 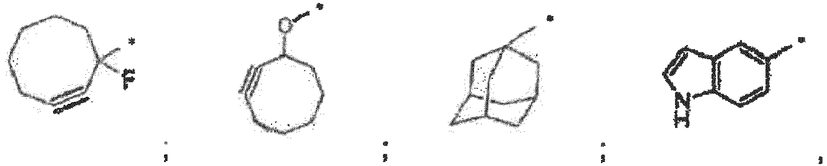
NXN représente un liant de diamine en C₁-C₈ linéaire ou cyclique ;
F représente un colorant fluorescent sélectionné parmi le groupe constitué de : 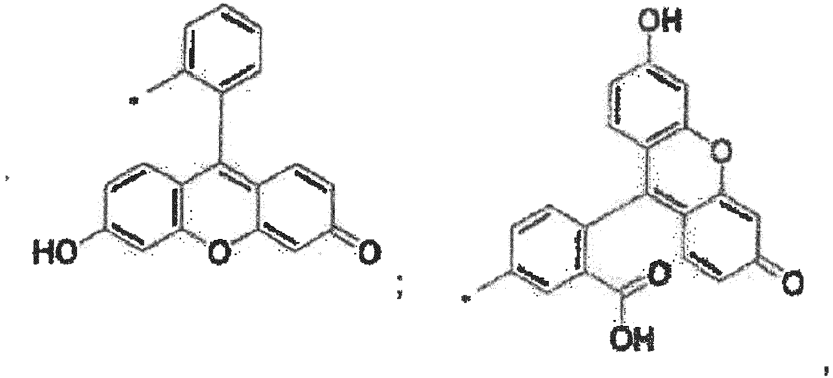 et
Y représente un écarteur en C₁-C₁₂ optionnel sélectionné parmi le groupe constitué d'un groupe caractéristique aliphatique substitué ou non substitué, d'un groupe caractéristique aromatique substitué ou non substitué ou d'une combinaison de ceux-ci.

2. Composition pharmaceutique destinée à être utilisée selon la revendication 1, dans laquelle le liant de diamine NXN est sélectionné parmi le groupe constitué de :

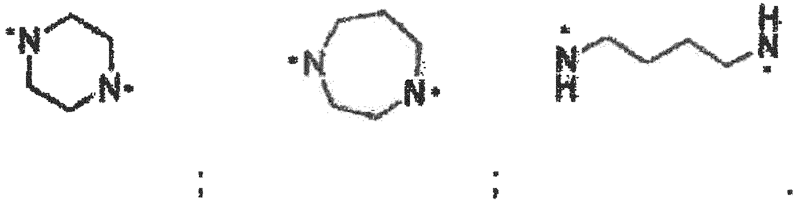

3. Composition pharmaceutique destinée à être utilisée selon la revendication 1 ou 2, dans laquelle le composé fluorescent est sélectionné parmi le groupe constitué de :

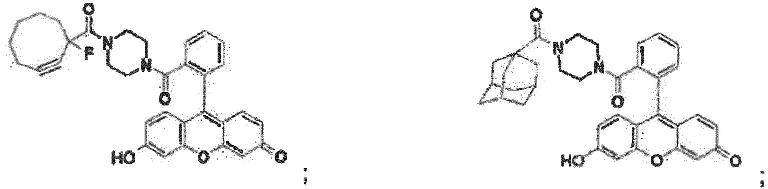

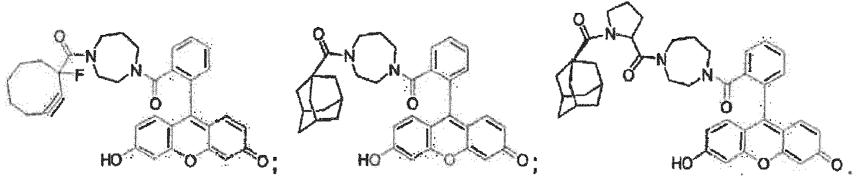

4. Composition pharmaceutique destinée à être utilisée selon l'une quelconque des revendications 1 à 3, dans laquelle la composition pharmaceutique comprend un excipient pharmaceutiquement acceptable et est un aérosol ou un baume.

5. Composition pharmaceutique destinée à être utilisée selon la revendication 4, dans laquelle la concentration en le composé fluorescent va de 0,1 µM à 100 µM.

6. Composition pharmaceutique destinée à être utilisée selon l'une quelconque des revendications 1 à 5, dans laquelle le procédé comprend les étapes consistant à :
(a) appliquer la composition pharmaceutique sur un tissu cutané qui est suspecté de contenir des cellules de mélanome malin ;
(b) laisser incuber le tissu cutané pendant une période 1 min à 6 h ;
(c) éliminer en option tout excès de composition pharmaceutique du tissu cutané ;
(d) irradier le tissu cutané en utilisant une source lumineuse d'excitation qui convient à exciter le composé fluorescent contenu dans la composition pharmaceutique ;
(e) examiner visuellement le tissu cutané ; et
(f) en fonction du signal de fluorescence obtenu à l'étape (e), déterminer la présence d'un mélanome malin dans le tissu cutané.

7. Composition pharmaceutique destinée à être utilisée selon la revendication 6, dans laquelle la source lumineuse d'excitation à l'étape (d) est une lampe UV habituelle.

8. Procédé ex vivo pour la détection d'un mélanome malin dans un tissu cutané, dans lequel le procédé comprend les étapes consistant à :
(a) appliquer la composition pharmaceutique selon l'une quelconque des revendications 1 à 5 sur un tissu cutané qui est suspecté de contenir des cellules de mélanome malin ;
(b) laisser incuber le tissu cutané pendant une période 1 min à 6 h ;
(c) éliminer en option tout excès de composition pharmaceutique du tissu cutané ;
(d) irradier le tissu cutané en utilisant une source lumineuse d'excitation qui convient à exciter le composé fluorescent contenu dans la composition pharmaceutique ;
(e) examiner visuellement le tissu cutané ; et
(f) en fonction du signal de fluorescence obtenu à l'étape (e), déterminer la présence d'un mélanome malin dans le tissu cutané.

9. Procédé ex vivo selon la revendication 8, dans lequel la source lumineuse d'excitation à l'étape (d) est une lampe UV habituelle.

10. Composé fluorescent pour la détection d'un mélanome malin, dans lequel le composé fluorescent est sélectionné parmi le groupe constitué de :
